# EUROPÄISCHE PATENTANMELDUNG

(11) **EP 0 026 252 A1**
(43) Veröffentlichungstag der Anmeldung: **08.04.1981**
(21) Anmeldenummer: 80100245.2
(22) Anmeldetag: 18.01.1980
(51) Int. Cl.: A61K 7/22

(54) **Mittel zur oralen Hygiene**

(30) Priorität: 27.09.1979 NO 793113
(71) Anmelder: Blendax-Werke R. Schneider GmbH & Co., D-6500 Mainz (DE)
(72) Erfinder: Rölla, Gunnar, Oslo 3 (NO)

(57) **Zusammenfassung**

Mittel zur oralen Hygiene mit einem Gehalt an Chlorhexidin bzw. Alexidin (und/oder deren Salzen), die als die Verfärbung des Mund- und Rachenraums inhibierenden Stoffe Zinksalze enthalten.

## Beschreibung

Die vorliegende Erfindung betrifft ein Mittel zur oralen Hygiene, das insbesondere der Belagsbildung auf menschlichen Zähnen entgegenwirkt, ohne die diesen Mitteln bisher anhaftenden Nebenwirkungen aufzuweisen.

Es ist bereits seit langem bekannt, daß 1,6-Di-4'-chlorphenyldiguanidohexan (Chlorhexidin) und 1,6-Di-(2-ethylhexyl)diguanidchexan (Alexidin) bzw. deren Salze geeignet sind, die Bildung von menschlichem Zahnbelag wirksam und anhaltend zu verhindern. Dies beruht mutmaßlich darauf, daß diese Substanzen gegen die für die Belagsbildung verantwortlichen Bakterien wirken und aufgrund ihrer Substantivität zu menschlichem Zahnschmelz auch eine länger anhaltende Wirkung entfalten.

Eine Übersicht über die diesbezüglichen Eigenschaften von Chlorhexidin findet sich im Journal of Periodontal Research, Suppl. Nr. 12, 1973, "Symposium on Chlorhexidinein the Prophylaxis of Dental Disease".

Die Wirksamkeit von Alexidin ist ebenfalls Gegenstand zahlreicher Veröffentlichungen, hier sei nur beispielhaft auf die DE-OS Nr. 1 964 196 verwiesen.

Dem breit gestreuten Einsatz dieser Verbinduncen in Zahn- und Mundpflegemitteln zur routinemäßigen täglichen Anwendung stand bisher, trotz ihrer unbestreitbaren einschlöqigen Wirksamkeit, die Nebenwirkung gegenüber, daß bei intensiver langanhaltender Anwendung von diese Substanzen enthaltenden Zusammensetzungen eine starke Verfärbung der Zähne und häufig auch der Zunge auftritt, vgl. beispielsweise Journal of Dental Rescarch, Vol. 79 (1971), S. 119 - 125.

Es wurde bereits vorgeschlagen, diese Verfärbung durch Verwendung wasserunlöslicher Salze des Chlorhexidins zu verhindern (DE-OS Nr. 2 158 102). Dieser Vorschlag ist jedoch insofern nicht realisierbar, als die dort beispielhaft angeführten Salze wie das Dihydrochlorid doch zu einem gewissen Teil wasserlöslich sind und auch wasserlöslich sein müssen, da ein vollkommen wasserunlösliches Salz keine Wirksamkeit aufveisen kann, und sich deshalb auch die eruähnten Nebenwirkungen nicht vermeiden lassen.

Aus der DE-OS Nr. 2 338 177 ist es ferner bereits bekannt, Chlcrhexidin und dessen Salze enthaltenden Zusammensetzungen Harnstoff zuzusetzen, um eine Verfärbung der Zähne zu verhindern; jedoch funktioniert auch dies nur bis zu einem gewissen Grad, darüberhinaus sind hierfür relativ hohe Harnstoffkonzentrationen erforderlich, die möglicherweise bei Zahnpasten bestimmter Zusammensetzung Stabilitätsprobleme aufwerfen können.

Auch die US-PS Nr. 4 080 441 befaßt sich mit diesem Problem, dort wird zu dessen Lösung die Verwendung eines Bis-(o-carboxypheyl)esters einer C₂-C₈-aliphatischen Dicarbonsäure empfohlen. Der Einsatz derartiger Verbindungen wirft in einem Mittel zur oralen Hygiene nicht nur geschmackliche Probleme auf, sondern führt auch zu keiner befriedigenden Wirkung.

Es wurde nun qefunden, daß man die Tendenz von Zahn- und Mundpflegemitteln, die Chlorhexidin oder Alexidin bzw. deren Salze enthalten, zur Verfärbung der Zähne dadurch verhindern kann, daß man diesen Zusammensetzungen ein wasserlösliches Zinksalz zusetzt.

Ohne im vorliegenden Fall den Reaktionsmechanismus der Erfindung einer verbindlichen Theorie unterwerfen zu wollen, könnte die die Verfärbung der Zähne in Gegenwart von Chlorhexidin- bzw. Alexidin-Salzen verhindernde Wirkung der Zinksalze darauf beruhen, daß auf den Zähnen bzw. der Schleimhaut ein dünner Einweißfilm gebildet wird, der in Gegenwart des antibakteriellen Mittels denaturiert wird und eine Verfärbung dadurch verursacht, daß die vorhandenen -SH- und -SS-Gruppen mit im Speichel durch die Nahrung zugeführten Eisenionen reagieren und dunkel gefärbte Eisensulfide bilden. Falls die Bildung dieser dunklen Verbindungen inhibiert wird, kann eine Verfärbung nicht auftreten. Durch den Zusatz von Zinksalzen, die Zinkionen bilden, kann dies nach dieser Theorie so verhindert werden, daß wasserunlösliche farblose oder weiße Sulfide, wie Zinknulfid, gebildet und auf der Zahnoberfläche niedergeschlagen werden,ohne dort sichtbar zu sein.

Dies würde also eine Abkehr von der bisherigen Theorie, daß die Verfärbung nach der Anwendung von Chlorhexidin- bzw. Alexidin-Salzen durch Retention dieser Verbindungen auf den Zahnoberflächen und ihre dunkel gefärbten Abbauprodukte veranlaßt wird, bedeuten.

Es sei jedoch nochmals betont, daß es sich hierbei lediglich um eine Theorie handelt, die den Schutzumfang in keiner Weise begrenzen kann und soll.

Geeignete Zinksalze im Rahmen der Erfindung sind alle toxikologisch anwendbaren, Zinkionen liefernden Verbindungen dieser Art wie Zinkchlorid, Zinksulfat, Zinknitrat, Zinkfluorid, Zinkfluorsilikat, Zinkchlorat, Zinkjodid, Zinkpermanganat, Zinkperoxid, organische wasserlösliche Zinksalze wie Zinkacetat, Zinklactat, Zincitrat, Zinkmalat, Zinkbutyrat, Zinkvalerat, Zinkcaproat, Zinklaurat, Zinkglycerophosphat, Zinkphenolsulfonat, Zinktartrat, Zinksalicylat, Zinkbenzoat, Zinkgluconat, etc.

Die Menge an Zinksalz liegt etwa zwischen 0,01 und 5,0 Gew.-% (berechnet auf Zn ) des erfindungsgemäßen Mittels.

Als Salze des Chlorhexidins bzw. Alexidins sind insbesondere das Gluconat, das Acetat und das Lactat geeignet, jedoch können auch weitere Salze wie das Dihydrochlorid, das Dihydrofluorid etc., vorzugsueise in Hengen zwischen 0,01 und 2,5 Gew.-% (berechnet auf frei Base) der Gesamtzusammensetzung, eingesetzt werden.

Das bevorzugte Verhältnis zwischen Chlorhexidin- bzw. Alexidin-Salz und Zinksalz liegt zwischen 1 : 10 und 10 : 1, vorzugsweise zwischen 1 4 und 4 : 1, insbesondere zwischen 1 : 2 und 2 : 1.

Die erfindungsgemäßen Mittel zur oralen Hygiene können in jeder bekannten und akzeptablen Form vorliegen.

Falls es sich für direkt vom Verbraucher anzuwendende Präparate handelt, sind Zahnpasten oder Zahngele, Mundwässer, Zahnpulver, Kaugummis, Dragees, Lutschbonbons, Kapseln, etc. besonders geeignet.

Es ist jedoch auch möglich, gegebenenfalls höher konzentrierte Präparate, die auch vom Zahnarzt verabreicht werden können, einzusetzen, die in Form von Lösungen, Gelen, Salben, Emulsionen, Suspensionen oder Sprühzusammensetzungen formuliert sein können.

Die erfindungsgemäßen Zusammensetzungen können in einer Phase Anvendung finden, es ist jedoch auch möglich, sogenannte Zweiphasenpräparate herzustellen, d.h., zunächst ein Mundwasser mit einem Gehalt an Chlorhexidin- oder Alexidin-salz zur Mundspülung zu verwenden und anschließend mit einem zinksalzhaltigen Mundwasser nachzuspülen bzw. umgekehrt. Derartige Zweikomponenten-Zusamensetzungen und die hierfür geeigneten Verpackungen sind aus dem Stand der Technik an sich bekannt.

Aus der US-PS Nr. 4 022 880 sind bereits Zusammensetzungen zur Verhinderung der Zahnbelags- und Zahnstein-Bildung bekannt, die Zinkionen und ein antibakterielles Mittel in einem oral anwendbaren Träger enthalten. Unter zahlreichen möglichen antimikrobiellen Verbindungen sind auch Chlorhexidindigluconat und Chlorhexidindiacetat genannt, jedoch findet sich weder in den Beispielen noch sonst ein Hinweis auf diese Verbindungen, es stehen hier halogenierte Salicylanilide im Vordergrund. Darüberhinaus erfolgt dort die Mitverwendung einer Zinkverbindung nicht zur Verhinderung der Verfärbung durch Chlorhexidin- oder Alexidin-Salze,sondern zur synergistischen Wirkungssteigerung der aufgeführten antimikrobiellen Mittel, so daß zum Gegenstand der vorliegenden Erfindung keinerlei Berührung besteht. Eine Verhinderung der Verfärbung wäre bei den vorzugsweise Salicylanilide bzw. Phenolderivate als antimikrobielle Verbindungen enthaltenden Zusammensetzungen nach der US-PS 4 022 880 auch gar nicht erforderlich, da diese eine entsprechende Nebenwirkung nicht aufweisen. Andererseits sind diese Verbindungen hinsichtlich der belagsverhindernden Wirkung den Chlorhexidin- bzw. Alexidinsalzen weit unterlegen und auch aus toxikologischen Gründen für den längerdauernden Einsatz in der oralen Hygiene weniger geeignet.

Im folgenden wird anhand von Vergleichsversuchen die verfärbungsverhindcrnde Wirkung des Zusatzes von Zinksalzen zu Chlorhexidin- bzw. Alexidinsalzlösungen unter Beweis gestellt:

### Vergleichsversuch A

Eine Gruppe von 10 Testpersonen führte eine tägliche tlundspülung mit einer wäunrig alkoholischen Lösung von 0,2 % Chlorhexidindiacetat während aines Zeitrang von zwei Wochen durch.Vor Beginn des Versuchs wurden ibre sorgfältig gereinigt, während der Versuchsdauer wurde keine sonstige orale Hygiene durchgeführt. Zum Ende des Versuchs wurden der Plaqueindex nach Silness und Löe (Acta Odontol. Scand. 22 (1964), S. 112 - 135) und die Verfärbung der Zähne nach folgendem Schema untersucht:
1. Keine Verfärbung.
2. Leichte Verfärbung ohne kosmetische Beeinträchtigung.
3. Verfärbung, die kosmetisch unzumutbar war.

Nach einer Pause von einer Woche wurde eine zweite Versuchsreihe durchgeführt, wobei zur Hundspülung eine analoge Lösung, die 0,2 % Chlorhexidindiacetat und 0,3 % Zinkacetat enthielt, eingesetzt wurde.

Die Resultate beider Versuchsreihen waren wie folgt:

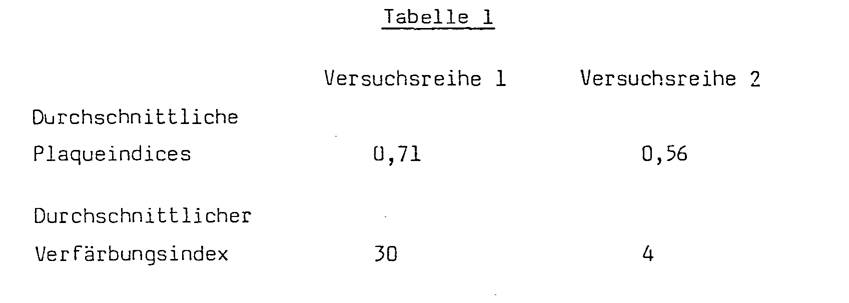

### Verqleichsversuch B

Fünf Patienten, die eine 0,2 %ige Lösung von Chlorhexidindiacetat zusätzlich zu ihrer normalen Mundhygiene täglich einmal benutzt haben, mußten aufgrund der eingetretenen Verfärbung einmal monatlich eine Reinigung der Zähne durch den Zahnarzt vornehmen lassen. Nachdem diesen Patienten eine identische Spüllösung verabreicht uurde, die zusätzlich 0,3 % Zinkchlorid enthielt, uurde nach einmonatiger Benutzungsdauer bei keinem der Patienten eine Verfärbung beobachtet, die eine Behandlung durch den Zahnarzt erforderlich gemacht hätte.

Die erfindungsgemäßen Mittel zur oralen Hygiene können weitere Wirkstoffe enthalten. Solche sind beispielsweise Fluorverbindungen, vorzugsweise in solchen Mengen, daß die Konzentration an reinem Fluor im Mittel etwa 0,01 bis etwa 1 Gew.-%, vorzugsucise 0,1 bis 0,5 Gew.-%, beträgt.

Geeignete Fluorverbindungen sind insbesondere die verschiedenen Salze der Monofluorphosphorsäure, insbesondere Natrium-, Kalium-, Lithium-, Calcium- und Aluminiummono- und-difluorphosphat, sowie die verschiedenen, Fluor in ionisch gebundener Form enthaltenden Fluoride, insbesondere Alkalifluoride wie Natrium-, Lithium-, Kalium- und Ammoniumfluorid, Zinnfluorid, Manganfluorid, Zirkoniumfluorid und Aluminiumfluorid sowie Gemische oder Anlagerungsprodukte dieser Fluoride untereinander oder mit anderen Fluorverbindungen,beispielsweise Kalium- oder Natriummanganfluorid.

Andere im Rahmen der vorlieqenden Erfindung mit einsetzbare Fluoride sind beispielsweise Zinkfluorid, Germaniumfluorid, Palladiumfluorid, Titanfluorid, Alkalifuorzirkonate,beispielsweise Natrium- oder Kaliumfluorosulfat.

Auch organische Fluorverbindungen können mit Erfolg eingesetzt werden, insbesondere die bekannten Additionsprodukte aus langkettigen Aminen oder Aminosäuren und Fluorwasserstoff, Monoäthanolaminohydrofluorid oder Methyltriäthylammoniumfluorid.

Die erfindungsgemäßen Mittel können weitere,zur Verwendung in solchen Mitteln an sich bekannte Stoffe enthalten, beispielsweise Enzyme wie Proteasen und Carbohydrasen wie Amylase, Dextranase, Lävanase oder α-1,3-Glucan-3-glu- canohydrolase oder Zahnsteinbildung bekämpfende Substanzen wie die für diesen Zueck vorgeschlagenen Phosphonsäuren, beispielsweise Hydroxyäthan-1,1- diphosphonsäure.

Eine ausführliche Übersicht über die Herstellung von Zahnpflegemitteln und die dabei zum Einsatz gelangenden Stoffe findet sich in dem Handbuch von M.S. Balsam und E. Sagarin, "Cosmeties-Science and Technology", 2nd Ed., Vol. 1, S. 423 bis 531 (1972).

Im folgenden werden Ausführungsbeispiele für geeignete Zusammensetzungen zur oralen Hygiene mit einem Gehalt an Chlorhexidin bzw. Alcxidinsalzen und Zinksalzen gegeben:

### Beispiel 1

Zahnpasta

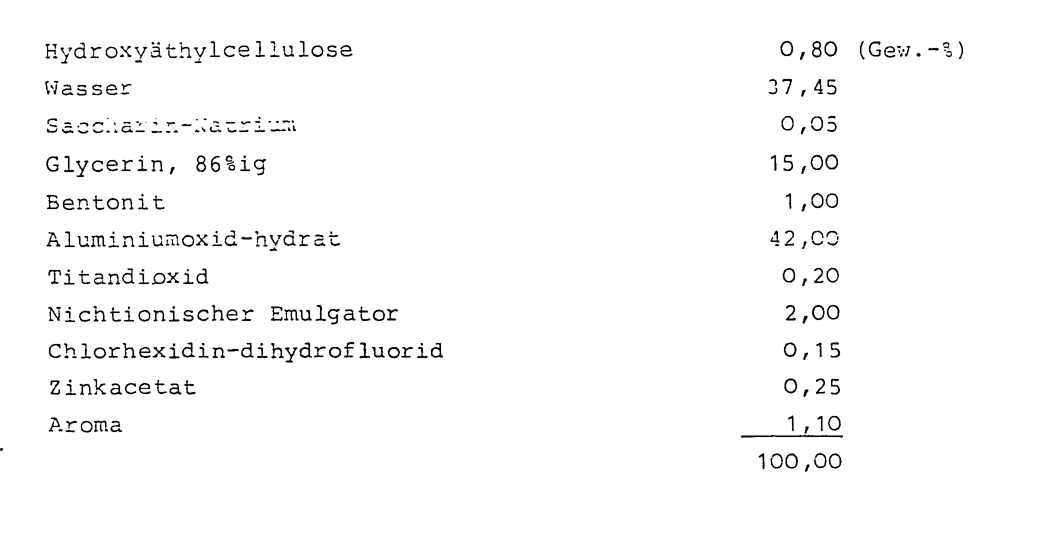

### Beispiel 2

Transparente Zahnpasta

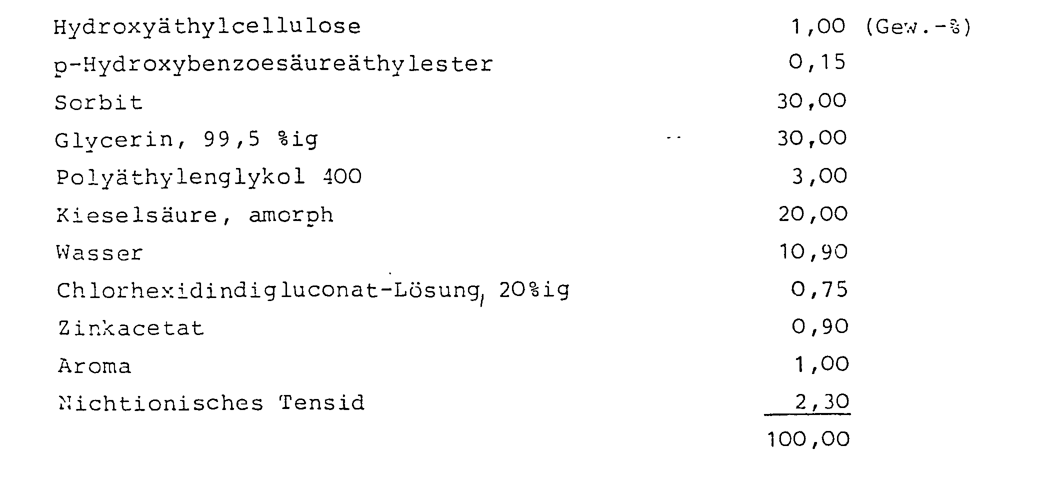

### Beispiel 3

In zwei Phasen vorliegende Zahnpasta (in einer Zweikammertube mit getrennten Öffnungen verpackt).

### Pasta A

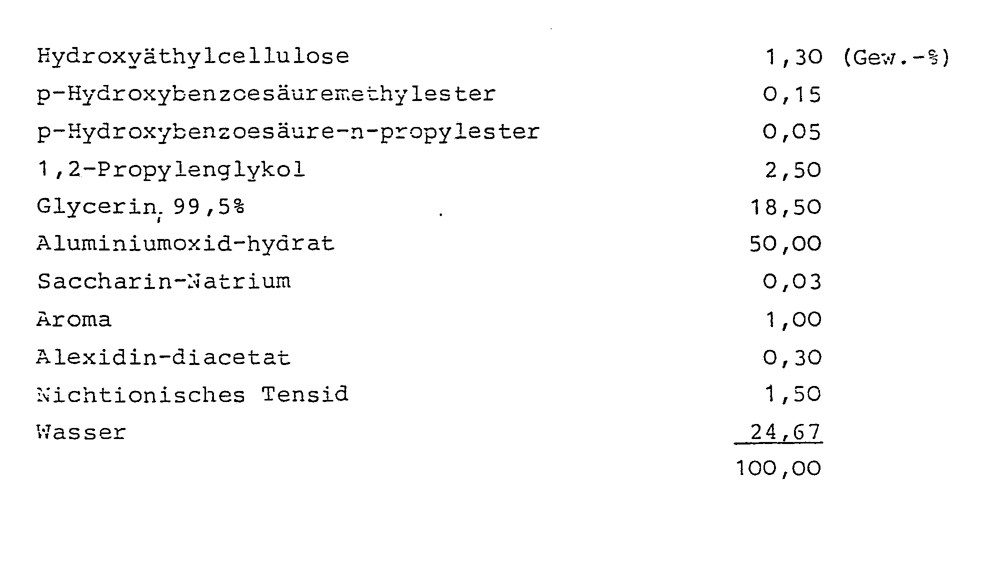

Pasta B

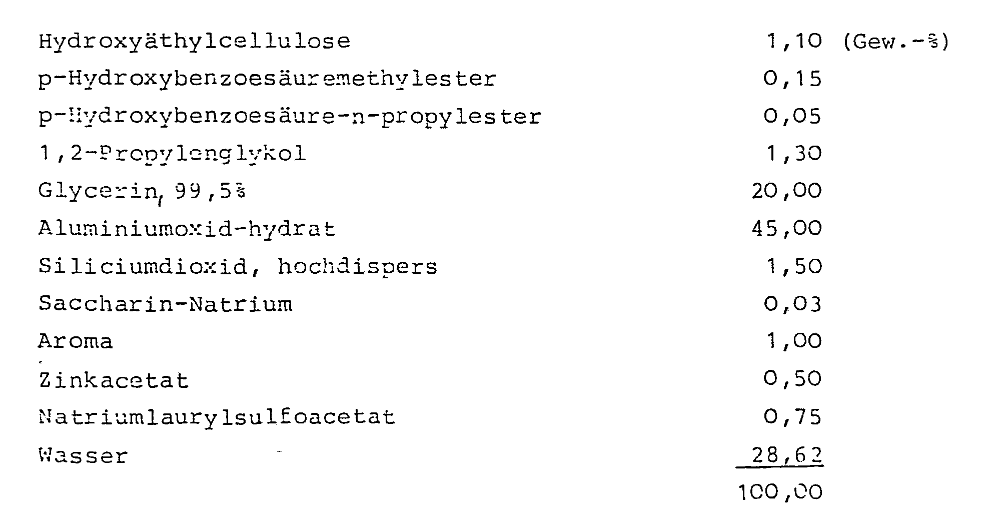

### Beispiel 4

Mundwasserkonzentrat

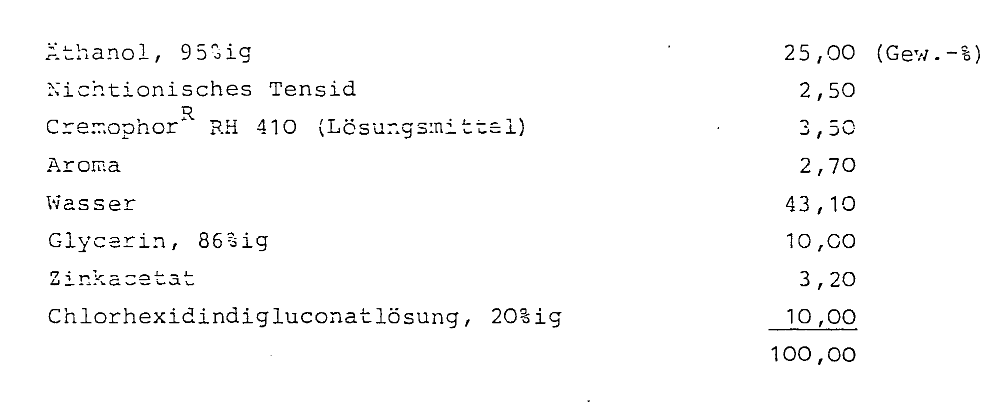

### Beispiel 5

Gebrauchsfertiges Mundwasser

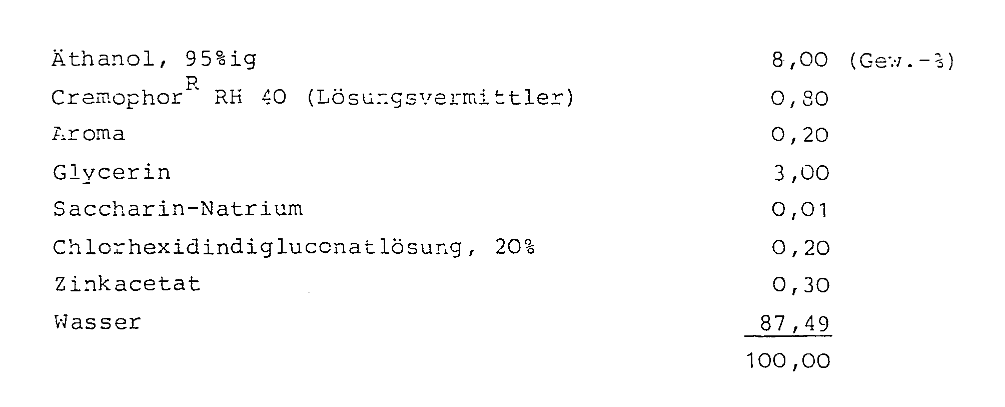

### Beispiel 6

Kaugummi

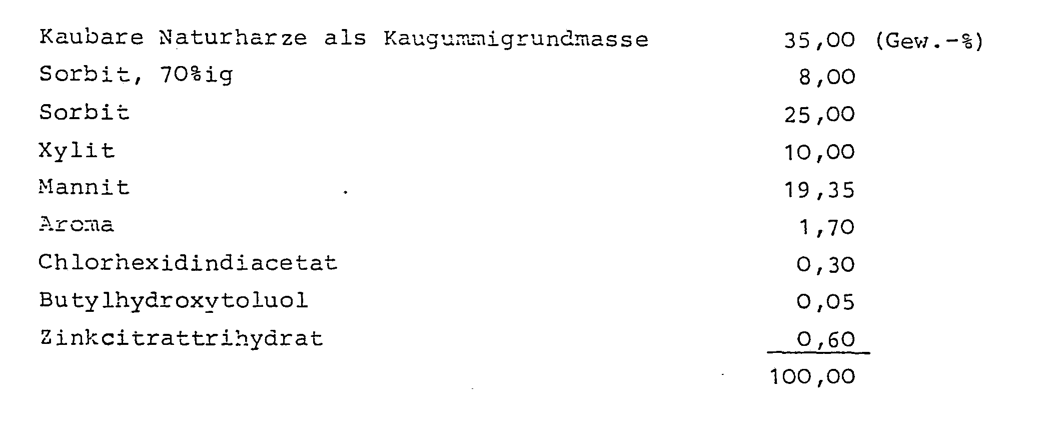

### Beispiel 7

Zweischichtenbonbon

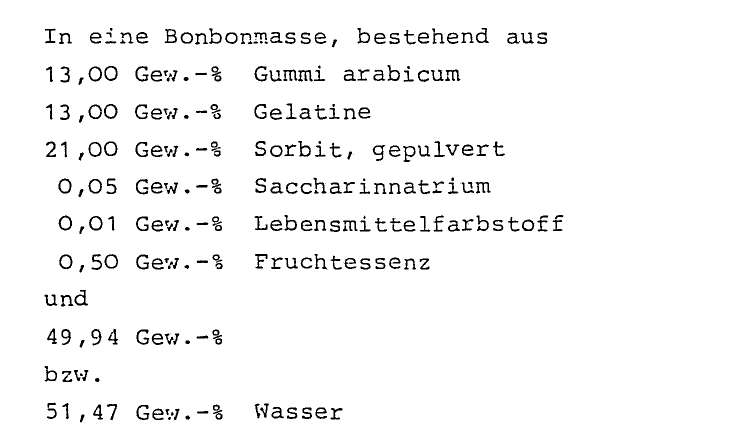

wurden einmal 0,50 Gew.-% Chlorhexidindigluconat und in einem getrennten Ansatz
1,80 Gew.-% Zinkacetat
eingarbeitet und in üblicher Weise gegebenenfalls verschiedenfarbige Bonbons ausgeformt. Jeweils zwei Bonbons mit verschiedenem Wirkstoffgehalt wurden zu einem "Benbonpaar" verpackt.

### Beispiel 8

Gefüllte Kapsel

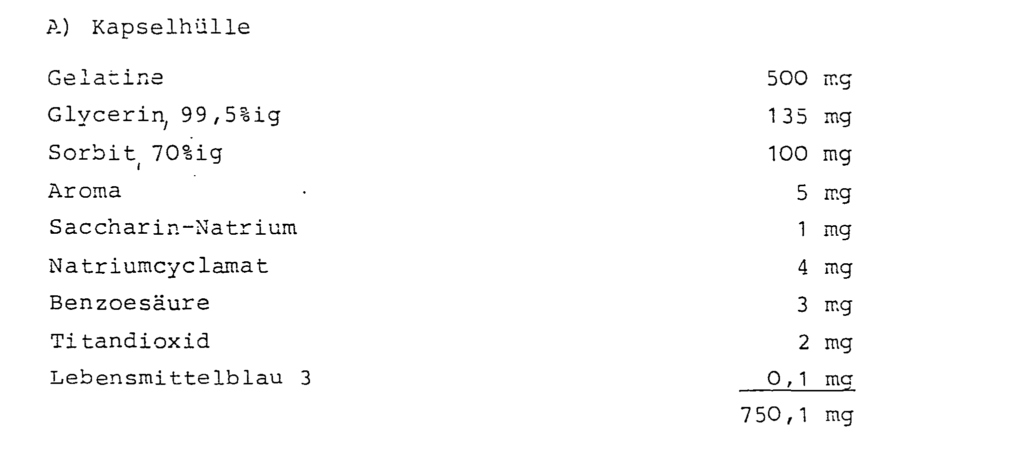

b) Füllung

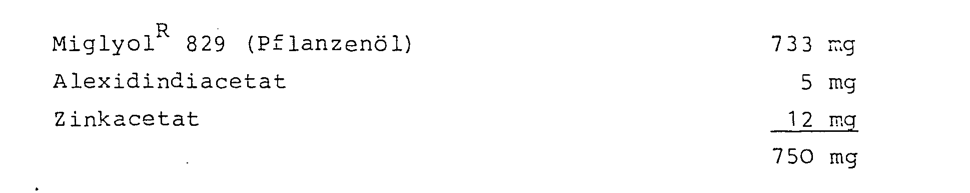

Eine Kapsel enthält 5 mg Alexidindiacetat und 12 mg Zinkacetat.

Unter den erfindungsgemäßen Mitteln zur oralen Hygiene sind auch Mittel zur Behandlung von Zahnprothesen zu verstehen, was durch das folgende Ausführungsbeispiel dokumentiert wird:

### Prothesenreinigungstablette

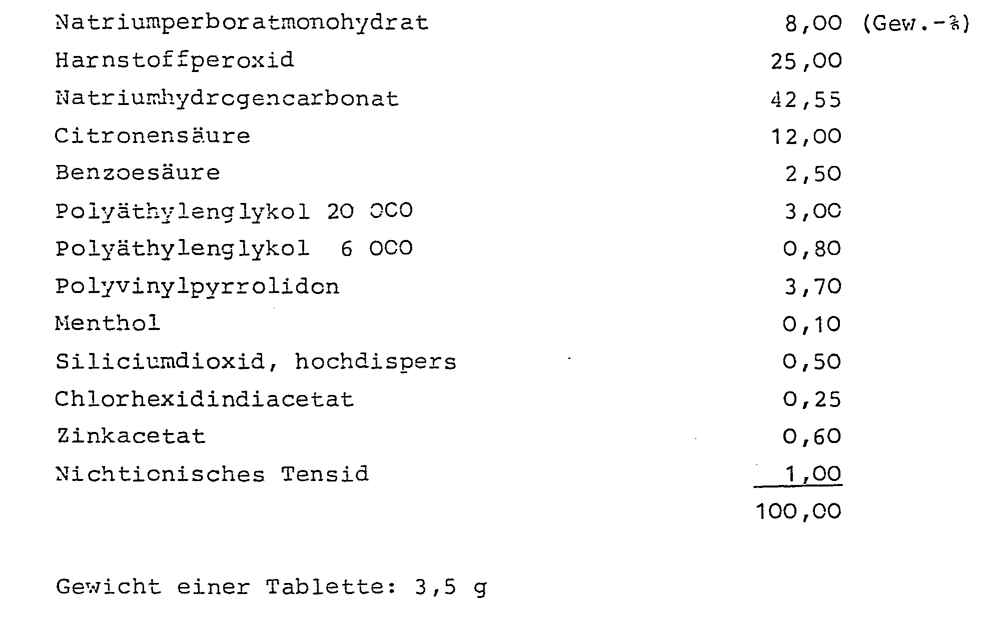

## Patentansprüche

Mittel zur oralen Hygiene auf Basis der üblichen Träger- und Zusatzstoffe mit einem Gehalt an 1,6-Di-4'-(chlorphenyldigua- nido)hexan und/oder 1,6-Di-(2-ethylhexyldiguanido)hexan bzw. deren Salzen, gekennzeichnet durch einen Gehalt von 0,01 bis 5 Gew.-% (berechnet auf Zn) eines oder mehrerer wasserlöslicher Zinksalze.
